(19) **Europäisches Patentamt / European Patent Office / Office européen des brevets**

(11) **EP 1 782 776 A1**

(12) **EUROPEAN PATENT APPLICATION**
published in accordance with Art. 158(3) EPC

(43) Date of publication:
    **09.05.2007  Bulletin 2007/19**

(51) Int Cl.:
    **A61F 7/08** (2006.01)   **C09K 5/16** (2006.01)
    **F24J 1/00** (2006.01)

(21) Application number: **05765769.4**

(22) Date of filing: **14.07.2005**

(86) International application number:
    **PCT/JP2005/013005**

(87) International publication number:
    **WO 2006/006652 (19.01.2006 Gazette 2006/03)**

(84) Designated Contracting States:
    **DE FR GB**

(30) Priority:  **14.07.2004  JP 2004207833**

(71) Applicant: **Mycoal Products Corporation**
    **Tochigi-chi, Tochigi 328-0067 (JP)**

(72) Inventor: **DODO, Toshihiro,**
    **c/o MYCOAL PRODUCTS CORPORATION**
    **Tochigi-shi, Tochigi, 328 067 (JP)**

(74) Representative: **Thun, Clemens**
    **Mitscherlich & Partner**
    **Sonnenstrasse 33**
    **80331 München (DE)**

(54) **HEATING ELEMENT**

(57)    To provide a heat generating body in which a space can be surely secured in the surroundings of a heat generating composition and heat insulation of the heat generating composition, adjustment of the quantity of air to be taken in and prevention of dispersion of water can be achieved.

A heat generating body having an exothermic part having a heat generating composition molded body which is a molded body of a heat generating composition capable of causing heat generation upon contact with air in a convex of an accommodating bag having plural irregularities by laminating the heat generating composition molded body on a substrate, covering a covering material thereon and then heat sealing the periphery of the heat generating composition molded body, **characterized in that** the heat generating composition contains, as essential components, an exothermic substance, a carbon component, a reaction accelerator and water; that the heat generating body is composed of a sectional exothermic part and a sectioned part, the sectional exothermic part accommodates the heat generating composition molded body therein, the sectioned part is a seal part, and the sectional exothermic part is disposed via the sectioned part; that at least a part of the sectional exothermic part has an air-permeable surface; that the air-permeable surface which comes into contact with the heat generating composition molded body is constituted of a non-water absorptive raw material; that the sectional exothermic part and the sectioned part have a difference of altitude, and the part having a difference of altitude is covered by an air permeability adjusting material via a bonding layer and forms a partitioned spacial part; and that the spacial part is provided with a primary air intake.

FIG.2

EP 1 782 776 A1

## Description

[Technical Field]

The present invention relates to a heat generating body which equalizes and efficiently reveals expected reaction heat of a heat generating composition, makes the whole of an exothermic part of the heat generating body warm within a short period of time, controls the movement of a gas from the heat generating composition by an air permeability adjusting material and a spacial air-permeable layer, minimizes influences of a change of the outside air temperature, effectively achieves heat insulation of the heat generating composition to prevent a lowering of the temperature of the heat generating composition per se due to an inflow of cold outside air from occurring, uniformly achieves an exothermic reaction, eliminates going out of the exothermic reaction, is small in scattering of the temperature, achieves the heat generating over a long period of time, and is excellent in temperature characteristics.

[Background Art]

In the production of a heat generating body capable of causing heat generation due to the presence of air, the management of the exothermic temperature of the heat generating body has been carried out by managing the air permeability or moisture permeability of a packaging material.

In the heat generating body of such a kind, it is the present state that when by making at least one of a substrate and a covering material which constitute the packaging material porous, air permeability is brought and by managing this air permeability, the feed amount of air is controlled to achieve the temperature control.
So far, as a method for imparting air permeability to the substrate or covering material, the following methods have been proposed.

(1) In producing an air-permeable substrate or covering material, a synthetic resin-made film such as polyethylene-made resin films is laminated on a non-woven fabric such as nylon non-woven fabrics, the laminate is pricked with a pin, and the quantity of airflow is managed and controlled by the size of the pin or the number of pinholes, thereby controlling the exothermic speed.

(2) A fine powder such as calcium carbonate and barium sulfate is kneaded with a polyolefin based resin such as polyethylene resins, a polyolefin based resin-made film as obtained by molding this polyolefin based resin composition is stretched to generate cracks in the polyolefin based resin-made film to make it porous, thereby imparting air permeability, and a non-woven fabric such as nylon non-woven fabrics is stuck while managing the air permeability or moisture permeability.

In other words, according to this method, by controlling the stretching ratio of the polyolefin based resin-made film and the thickness of the non-woven fabric and further the sticking area between the polyolefin based resin-made film and the non-woven fabric, the air permeability or moisture permeability is managed.

There has also been made a proposal in which by using an air-permeable multilayered structure made of a combination of a non-woven fabric such as nylon non-woven fabrics with a perforated film or a porous film, a change in the sensing temperature to which a human body is sensitive with respect to a change in the outside air temperature is made small.

That is, in the heat generating body of such a kind, in making at least one of a substrate and a covering material which constitute the packaging material porous, it is the present state that air permeability is brought by providing perforations by a pin or performing processing such as stretching, the temperature control is performed by this air permeability.

Also, Patent Document 1 discloses a heat generating body having a heat generating composition capable of causing heat generation due to the presence of air accommodated in a flat packaging material made of a substrate and a covering material, in which an air-permeable layer is laminated on at least one or both of the substrate and the covering material in the side of the contact surface thereof with the heat generating composition and which is constituted such that the air flows into the inside from the end surface in the side of the periphery of this air-permeable layer via the air-permeable layer.

However, in the case where a powdered heat generating composition is accommodated in the packing material, leakage of the powder of this heat generating composition occurs to likely stain an underwear or the skin; and in opening holes in the substrate or covering material by pin perforation, the substrate or covering material expands and contracts, and as a result, the hole size at the time of hole opening is changed, the hole size is changed with a lapse of time, or a large scatter is caused in the substantial hole size depending upon the generation state of burrs, whereby not only the air permeability or moisture permeability is largely scattered, but also the management or control thereof is extremely difficult.

In the laminate of a non-woven fabric and a porous film, even if the stretching ratio of a polyolefin based resin-made film and the thickness of a non-woven fabric and further the sticking area between the polyolefin based resin-made film and the non-woven fabric is made uniform, a porous film having a uniform air permeability or moisture permeability is not always obtained. Much more, in a polyolefin based resin-made film as obtained by stretching, the shape and the distribution and further the size of cracks are largely different depending upon the place in the width direction and longitudinal direction, the control is very difficult, the stability is poor, and a large scatter in the air permeability or moisture permeability is generated. Thus, it is the present state that the management and control of the air permeability or moisture permeability, which is a very important management item in view of managing the exothermic temperature, is actually extremely difficult and that the width of the management is extremely largely scattered in the range of 20 to 30 % in the plus side and the minus side, respectively against an average value thereof. In order to make the standard width of the air permeability or moisture permeability narrower, a loss rate (20 to 30 % in the existing circumstances) becomes large, resulting in high costs, and nonstandard substrates or covering materials are incorporated. Thus, the quality management becomes remarkably troublesome.

In addition, in stretching the polyolefin based resin-made film, not only a special and expensive stretching device is needed, but also the number of steps increases and the productivity of a porous film is remarkably lowered, thereby rendering the production costs of the substrate or covering material extremely high.

Furthermore, with respect to the laminate of a perforated film, a non-woven fabric and a porous film, the air permeability or moisture permeability is largely scattered. Thus, there were involved various problems from the viewpoint of safety such that a required exothermic temperature is not obtained, an effective thermal effect is not obtained, and conversely, the temperature excessively rises, thereby causing a moderate-temperature burn.

Furthermore, in heat generating bodies which have been used from old, a substrate or a covering material is formed in a porous state, and air permeability is imparted to the entire surface. Accordingly, on the entire surface of the heat generating body, air flows in through air holes to the vertical direction or substantially vertical direction against the surface direction of the heat generating body, whereby the entire surface of the heat generating body causes an exothermic reaction at the same time. For that reason, not only scattering in the air permeability leads directly to scattering in the exothermic temperature, but also since the central part of the heat generating body is smaller in heat radiation than the surroundings thereof, the temperature in the central part of the heat generating body rises as compared with that in the surroundings, resulting in an increase of danger of a moderate-temperature burn. Thus, there was encountered a problem in view of putting it into practical use.

Then, up to date, a production technology or management technology of a packaging material capable of strictly managing the air permeability or moisture permeability of the packaging material has not been established yet and a scattering width is large so that a loss rate is high as 20 % to 30 %. Thus, there were involved problems in effective use of resources and costs. It is the present state that the management and control of the air permeability or moisture permeability is actually extremely difficult and that the width of the management is extremely largely scattered in the range of 20 to 30 % in the plus side and the minus side, respectively against an average value thereof. In order to make the standard width of the air permeability or moisture permeability narrower, a loss rate (20 to 30 % in the existing circumstances) becomes large, resulting in high costs, and nonstandard substrates or covering materials are incorporated. Thus, the quality management becomes remarkably troublesome.

In addition, in stretching the polyolefin based resin-made film, not only a special and expensive stretching device is needed, but also the number of steps increases and the productivity of a porous film is remarkably lowered, thereby rendering the production costs of the substrate or covering material extremely high.

With respect to the laminate of a perforated film, a non-woven fabric and a porous film, the air permeability or moisture permeability is largely scattered. Thus, there were involved various problems from the viewpoint of safety such that a required exothermic temperature is not obtained, an effective thermal effect is not obtained, and conversely, the temperature excessively rises, thereby causing a moderate-temperature burn.

However, according to the method as disclosed in Patent Document 1, air flows in to the horizontal direction or substantially horizontal direction against the surface direction of the heat generating body, and the exothermic reaction proceeds step by step inwardly from the surroundings of the heat generating body. Thus, though the duration of the heat generating body can be prolonged, the rising time as a heat generating body becomes slow, and a temperature difference between the surroundings and the central part of the heat generating body is large at the beginning so that an uncomfortable feeling is brought. In addition, since the temperature distribution horizontally moves with a lapse of time, warmth taking continues without overcoming the temperature difference over a long period of time. Although the heat generating body has an effect such that the same place is not heated at a peak (high) temperature over a long period of time, a phenomenon in which the surroundings of the heat generating body are cold because of completion of the reaction whereas the central part is warm occurs. Thus, this is problematic in view of putting it into practical use as a heat generating body.

Furthermore, as it becomes closed to the central part of the heat generating body, the quantity of inflow of air is lowered due to resistance. Thus, as the transfer area of the heat generating composition becomes large, what the exothermic temperature in the central part of the heat generating body is lowered creates discomfort at the time of use.

In addition, air flows in to the horizontal direction or substantially horizontal direction against the surface direction of the heat generating body; an exothermic reaction proceeds step by step inwardly from the circumference of the heat generating body; with respect to the temperature rise, it takes a time to spread the heat generation into the central part as compared with a porous film, namely an air-permeable film in which air permeability is imparted over the entire surface; the temperature rise continues only in the surroundings; and a low temperature continues as a whole. Thus, dissatisfaction leaves in a feeling of temperature. While it is easy to drop the temperature, it is difficult to raise the temperature, and therefore, the utility is limited.

That is, in the heat generating body of such a kind, in making at least one of a substrate and a covering material which constitute the packaging material porous, it is the present state that air permeability is brought by providing perforations by a pin or performing processing such as stretching, the temperature control is performed by this air permeability.

Furthermore, the dispersion of water from a heat generating composition which starts from the start of heat generation was substantially untouched. The water is dispersed through the entire surface of the air-permeable surface, a reaction of an exothermic substance is not sufficiently carried out, a stoichiometric reaction of the exothermic substance is not completed, and the exothermic substance cannot fulfill its destiny. Such fell far short of the original exothermic duration of the heat generating body.

Furthermore, usually, the exothermic reaction of a chemical body warmer utilizing oxidation reaction heat of an iron powder naturally varies depending upon the outside air circumferential temperature. In order to minimize the influences by the outside air temperature, by making the most use of an air layer of the packaging material and feeding the taken air into an exothermic agent while warming, namely by using a non-woven fabric having a strength during the use and having a specific thickness and a specific volume ratio and feeding the air in parallel to the surface on which an exothermic agent is provided (horizontal air permeability), it was attempted to improve heat insulating properties, heat conducting properties, heat retaining properties and a touch (softness) of the body warmer. However, such was insufficient. In particular, in the case where the body warmer has a sectional exothermic part, the surface area increases. Thus, there was a limit in control.

Furthermore, in a heat generating body which is flat, is constituted of a single exothermic part and is formed of an air-permeable sheet having a number of air holes provided in the central part of the surface layer by perforation, it becomes properly warm immediately after the start of use. However, it involves the following problems.

(1) In the air holes, the temperature rapidly increases too much at the beginning.
(2) Movement with time of the exothermic site and sites having a different temperature are generated.
(3) Since air comes in only from the air holes, there is a possibility that a heat generating body in the end of a bag leaves without being utilized.
(4) Since the heat generating body is leaked from the air holes, it is necessary to use an excipient for the purpose of preventing the leakage from occurring. Since the excipient hardens the heat generating body itself, the problem as set forth above in (2) more likely takes place.

Furthermore, in a heat generating body which is flat, is constituted of a single exothermic part and is formed of an air-permeable sheet having different air permeability and made of a highly air-permeable part and a lowly air-permeable part, though it becomes properly warm immediately after the start of use, movement with time of the exothermic site and sites having a different temperature are generated. In addition, since the exothermic part is constituted of a single part, the fitness to curved surface parts of the body is poor, and a feeling for use is problematic. Moreover, in making the heat generating body large in size, a rough feeling increases. Thus, the feeling for use became deteriorated.

[Patent Document 1] JP-A-2000-260

[Disclosure of the Invention]

[Problems that the Invention is to Solve]

Then, an object of the invention is to provide a heat generating body in which a space can be surely secured in the surroundings of a heat generating composition and heat insulation of the heat generating composition, adjustment of the quantity of air to be taken and prevention of dispersion of water can be achieved.

[Means for Solving the Problems]

In order to solve these problems, the invention has been completed and is concerned with a heat generating body which is constituted of a spacial region constituted of an air-permeable raw material and a weakly air-permeable raw material or an air-impermeable raw material and connecting to an external section and an air vent as annexed thereto while utilizing a difference of altitude between a sectional exothermic part and a sectioned part and which is constituted such that air flows into the spacial air-permeable layer from the periphery of the sectional exothermic part. Specifically, as set forth in claim 1, a heat generating body of the invention is a heat generating body having an exothermic part having a heat generating composition molded body which is a molded body of a heat generating composition capable of causing heat generation upon contact with air accomodated in a convex of an accommodating bag having plural irregularities by laminating the heat generating composition molded body on a substrate, covering a covering material thereon and then heat sealing the periphery of the heat generating composition molded body, which is characterized in that:

1) the heat generating composition contains, as essential components, an exothermic substance, a carbon component, a reaction accelerator and water,
2) the heat generating body is composed of a sectional exothermic part and a sectioned part, the sectional exothermic part accommodates the heat generating composition molded body therein, the sectioned part is a seal part, and the sectional exothermic part is disposed via the sectioned part,
3) at least a part of the sectional exothermic part has an air-permeable surface,
4) the air-permeable surface which comes into contact with the heat generating composition molded body is constituted of a non-water absorptive raw material,
5) the sectional exothermic part and the sectioned part have a difference of altitude, and the sectional exothermic parts are covered with an air permeability adjusting material to forms a partitioned spacial part between the sectional exothermic parts, and
6) the spacial part is provided with a primary air intake.

Also, a heat generating body as set forth in claim 2 is

characterized in that in the heat generating body as set forth in claim 1, air permeability of the air permeability adjusting material is not more than that of the exothermic part.

Also, a heat generating body as set forth in claim 3 is characterized in that in the heat generating body as set forth in claim 1, the heat generating composition has a water mobility value of from 0.01 to 20.

Also, a heat generating body as set forth in claim 4 is

characterized in that in the heat generating body as set forth in claim 1, the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Also, a heat generating body as set forth in claim 5 is characterized in that in the heat generating body as set forth in claim 1, the spacial part has a spacial shape of at least one kind selected from a linear shape, a curved shape and a maze-like shape.

Also, a heat generating body as set forth in claim 6 is characterized in that in the heat generating body as set forth in claim 1, the air permeability adjusting material is an air-impermeable raw material.

Also, a heat generating body as set forth in claim 7 is characterized in that in the heat generating body as set forth in claim 6, the air permeability adjusting material is made of at least one kind selected from a thermoplastic synthetic resin film, a metal thin film-containing thermoplastic synthetic resin film, an air-impermeable multilayered structure having a laminate of a non-woven fabric and the thermoplastic synthetic resin film, a synthetic resin expanded body, and a multilayered structure containing the same.

Also, a heat generating body as set forth in claim 8 is characterized in that in the heat generating body as set forth in claim 1, the sectional exothermic parts are provided in a striped state at intervals interposing the sectioned part therebetween.

Also, a heat generating body as set forth in claim 9 is

characterized in that in the heat generating body as set forth in claim 1, the air intake is present in the surroundings of the heat generating body, and air present in the external space flows into the heat generating composition via the air intake.

Also, a heat generating body as set forth in claim 10 is characterized in that in the heat generating body as set forth in claim 1, the air intake is provided by perforating the air permeability adjusting material.

Also, a heat generating body as set forth in claim 11 is characterized in that in the heat generating body as set forth in claim 1, a space crossing the sectional exothermic parts is provided between the air permeability adjusting material and the exothermic part.

Also, a heat generating body as set forth in claim 12 is

characterized in that in the heat generating body as set forth in claim 11, the space crossing the sectional exothermic parts is provided in the substantially central part on the air-permeable surface.

Also, a heat generating body as set forth in claim 13 is characterized in that in the heat generating body as set forth in claim 1, the sectional exothermic part has a shortest length of from 5 to 200 mm and a maximum height of from 0.1 to 10 mm, a plural number of the sectional exothermic parts are disposed at intervals, and a gathered exothermic part is formed of a gathering of the sectional exothermic parts.

Also, a heat generating body as set forth in claim 14 is characterized in that in the heat generating body as set forth in claim 1, an air-impermeable film is provided so as to cover the air intake in a state that it can be peeled away.

Also, a heat generating body as set forth in claim 15 is characterized in that in the heat generating body as set forth in claim 1, a fixing measure is provided on a part of the exposed surface of the heat generating body.

Also, it is preferable that in the heat generating body, the air permeability adjusting material is welded via a bonding layer.

Also, it is preferable that in the heat generating body, the heat generating composition at least contains a component resulting from subjecting a mixture containing, as essential components, an iron powder, a carbon component, a reaction accelerator and water to a contact treatment with an oxidizing gas.

Also, it is preferable that in the heat generating body, the iron powder is covered by an iron oxide film on at least a part of the surface thereof; that the iron oxide film has a thickness of 3 nm or more; and that from 20 to 100 % by weight of an active iron powder having a region of an oxygen-free iron component is contained in at least one region selected from a central part region of the iron powder and a region beneath of the iron oxide film.

Also, it is preferable that in the heat generating body, the iron powder is covered by a wustite film on at least a part of the surface thereof; and that from 20 to 100 % by weight of an active iron powder having an amount of wustite of from 2 to 50 % by weight in terms of an X-ray peak intensity ratio to iron is contained.

Also, it is preferable that in the heat generating body, at least the heat generating composition molded body is compressed.

Also, it is preferable that in the heat generating body, the heat seal part is formed by heat sealing after temporary adhesion by an adhesive layer; and that an adhesive component constituting the adhesive layer and a component of a heat seal material constituting the heat seal layer are co-present in the heat seal layer.

Also, it is preferable that in the heat generating body, after heat sealing, at least a part of the accommodated heat generating composition molded body is moved into a temporary adhering part which is not heat sealed, thereby deadhering the temporary adhering part.

Also, it is preferable that in the heat generating body, the fixing measure is an adhesive layer; and that the adhesive layer contains at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous substance, a moisturizer, a functional substance, and a mixture thereof.

[Advantages of the Invention]

In the light of the above, according to the invention, by constituting a spacial air-permeable layer of an air permeability adjusting material and an air-permeable layer which comes into intimate contact with a heat generating composition, it is possible to surely secure a space in the surroundings of the heat generating composition. Thus, it has become possible to achieve heat insulation of the heat generating composition, adjustment of the quantity of air to be taken in and prevention of dispersion of water. That is, the invention brings the following advantages.

1. By providing a heat generating composition as two or more plural separated sectional heat generating compositions at intervals and further providing a first air-permeable layer such that it comes into contact with the sectional heat generating compositions, it is constituted that the plural sectional exothermic parts are present interposing a sectioned part therebetween, and concave-convex parts in which the fixed sectional heat generating compositions form a convex are alternately present. By combining this concave with the air permeability adjusting material, it has become possible to hold a sure space.

2. By making a space present along the heat generating composition, the whole of the heat generating composition enables an exothermic reaction to start simultaneously with breathing. Thus, it is possible to warm from the periphery to the central part of a heat generating body at the same time without causing unevenness of temperature.

3. By putting air intakes together and combining them with a spacial air-permeable layer, it is possible to prevent excessive dispersion of water in the heat generating composition and to obtain heat generation over a long period of time.

4. By adjusting an air permeability adjusting material and the quantity of opening of an air vent as annexed thereto, it becomes possible to adjust taking in of outside air by utilizing a difference in pressure between the spacial air-permeable layer and the outside air. When the reaction is vigorous, the space of the spacial air-permeable layer becomes in a state of reduced pressure, and the air permeability adjusting material is drawn to the side of an air-permeable raw material (heat generating composition). As a result, the space becomes small, the air vent becomes small, and the quantity of air which flows into the sectioned air vent becomes small, whereby the reaction stagnates. When the reaction stagnates, a degree of vacuum becomes low, and the air vent becomes large. For that reason, the quantity of inflow of air increases, and the reaction becomes again vigorous. By repetition of this, the quantity of inflow of air is in inverse proportion to the degree of reaction; the air permeability adjusting material works as a control valve of inflow of air; taking in of air becomes stable; the heat generation becomes uniform; and scattering of the temperature becomes remarkably small.

5. By constituting the spacial air-permeable layer of a space, it is possible to provide a heat generating body which achieves heat insulation/retention of a heat generating composition, equalizes and efficiently reveals expected reaction heat of the heat generating composition, makes the whole of an exothermic part of the heat generating body warm within a short period of time, controls the movement of a gas from the heat generating composition by a spacial air-permeable layer, minimizes influences of a change of the outside air temperature, effectively achieves heat insulation of the heat generating composition to prevent a lowering of the temperature of the heat generating composition per se due to an inflow of cold outside air from occurring, uniformly achieves an exothermic reaction, eliminates going out of the exothermic reaction, achieves the heat generating over a long period of time, and is excellent in temperature characteristics.

6. If desired, by providing a second spacial air-permeable layer extending over the sectional exothermic parts, it is possible to further improve the heat insulation of the heat generating composition, the uniformity of heat generation and the prevention of dispersion of water.

7. Since an excessive reaction of the gathered exothermic part is controlled, there is brought a combination of an effect for sufficiently achieving heating and heat insulation of a warmth-taking part which comes into contact with a human body or the like with an effect for controlling excessive heating in the gathered exothermic part.

8. By using, as the air permeability adjusting material, an adiabatic body such as heat insulating sheets resulting from vapor deposition of aluminum, silicon oxide, etc. on a synthetic resin film and expanded bodies, a heat insulating effect is enhanced, and far infrared rays emitted by the heat generating composition are reflected in the side of the heat generating composition, whereas the cold air is reflected in the reflection side, thereby achieving protection against the cold and heat insulation of the heat generating composition. Thus, going out of the reaction of the heat generating composition is prevented, and nevertheless a thin heat generating body, the warmth is obtained over a long period of time. [Best Modes for Carrying Out the Invention]

The invention is concerned with a heat generating body having an exothermic part having a heat generating composition molded body which is a molded body of a heat generating composition capable of causing heat generation upon contact with air in a convex of an accommodating bag having plural irregularities by laminating the heat generating composition molded body on a substrate, covering a covering material thereon and then heat sealing the periphery of the heat generating composition molded body, which is characterized in that:

1) the heat generating composition contains, as essential components, an exothermic substance, a carbon component, a reaction accelerator and water,
2) the heat generating body is composed of a sectional exothermic part and a sectioned part, the sectional exothermic part accommodates the heat generating composition molded body therein, the sectioned part is a seal part, and the sectional exothermic part is disposed via the sectioned part,
3) at least a part of the sectional exothermic part has an air-permeable surface,
4) the air-permeable surface which comes into contact with the heat generating composition molded body is constituted of a non-water absorptive raw material,
5) the sectional exothermic part and the sectioned part have a difference of altitude, and the part having a difference of altitude is covered by an air permeability adjusting material via a bonding layer and forms a partitioned spacial part, and
6) the spacial part is provided with a primary air intake.
The air permeability adjusting material controls air permeability and breathing passage into the exothermic part. Furthermore, the air permeability of a raw material which constitutes the air permeability adjusting material is not limited so far as it is possible to adjust the air accumulation or breathing. However, the air permeability is preferably not more than that of the air-permeable surface of the exothermic part.
Furthermore, the position of the air hole is not limited, but the air hole may be position in a central part, a spacial part or an end part.

Next, the embodiments of the invention will be described with reference to the accompanying drawings.

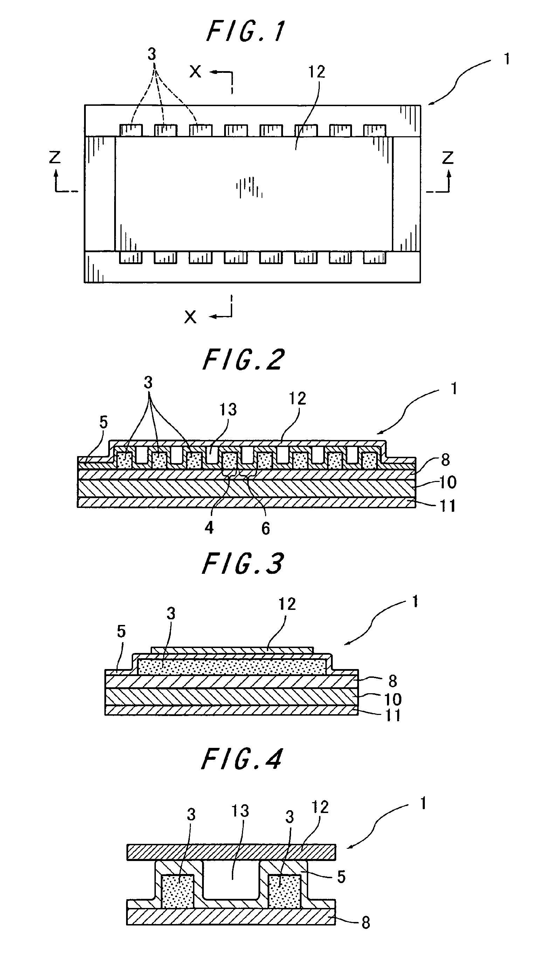

Fig. 1 is a plan view of the heat generating body of the invention; and Fig. 2 is a cross-sectional view along the line Z-Z of Fig. 1.
In a heat generating body 1 as illustrated in the drawings, a heat generating composition molded body 3 is laminated on an air-impermeable substrate 8, and the heat generating composition molded body 3 is covered by an air-permeable covering material 5. Incidentally, the substrate 8 is a laminate of a polyethylene-made non-woven fabric on an air-impermeable polyethylene film, and a separator 11 is provided beneath the substrate 8 via an adhesive 10. Furthermore, the covering material 5 is a laminate of a polypropylene-made non-woven fabric on a polyethylene-made porous film.

In the heat generating body 1, a sectional exothermic part 4 where the heat generating composition molded body 3 is present and a sectioned part 6 where the heat generating composition molded body 3 is not present are present. In the sectioned part 6, the covering material 5 and the substrate 8 are stuck to each other. Furthermore, in the periphery of the heat generating body 1, the substrate 8 and the covering material 5 are stuck to each other, too.

Furthermore, an air permeability adjusting material 12 is provided so as to cover a top of an adjacent heat generating composition molded body 3; and a spacial air-permeable layer 13 which is substantially a space is formed between a lower side of the air permeability adjusting material 12 and the sectioned part 6, an end part of which works as an air intake.

Fig. 3 shows a cross-sectional view along the line X-X of Fig. 1. Fig. 4 is an enlarged view in the vicinity of the spacial air-permeable layer 13 of Fig. 2.

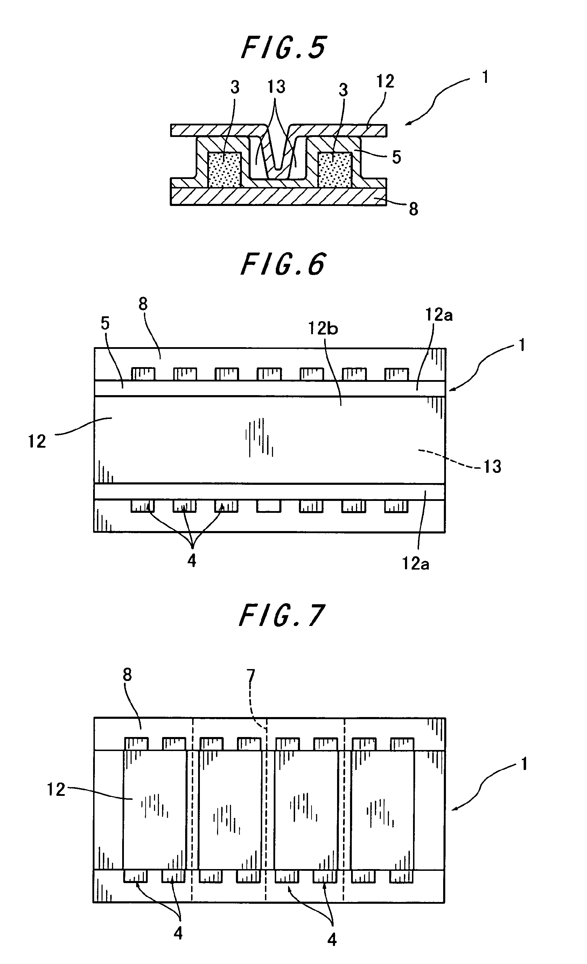

Fig. 5 shows an example in which two spacial air-permeable layers 13 are provided by sticking the air permeability adjusting material 12 to the covering material 5 in the substantially central part of the sectioned part 6 between the adjacent heat generating composition molded bodies 3.

Fig. 6 shows an example of using the air permeability adjusting material 12 provided with bonding layers 12a, 12a exclusive of a central part 12b of the air permeability adjusting material 12 of Fig.1.

Fig. 7 shows an example in which in Fig. 1, a perforation 7 is provided such that one pair of adjacent sectional exothermic parts 4, 4 can be divided as the sectioned part 6.

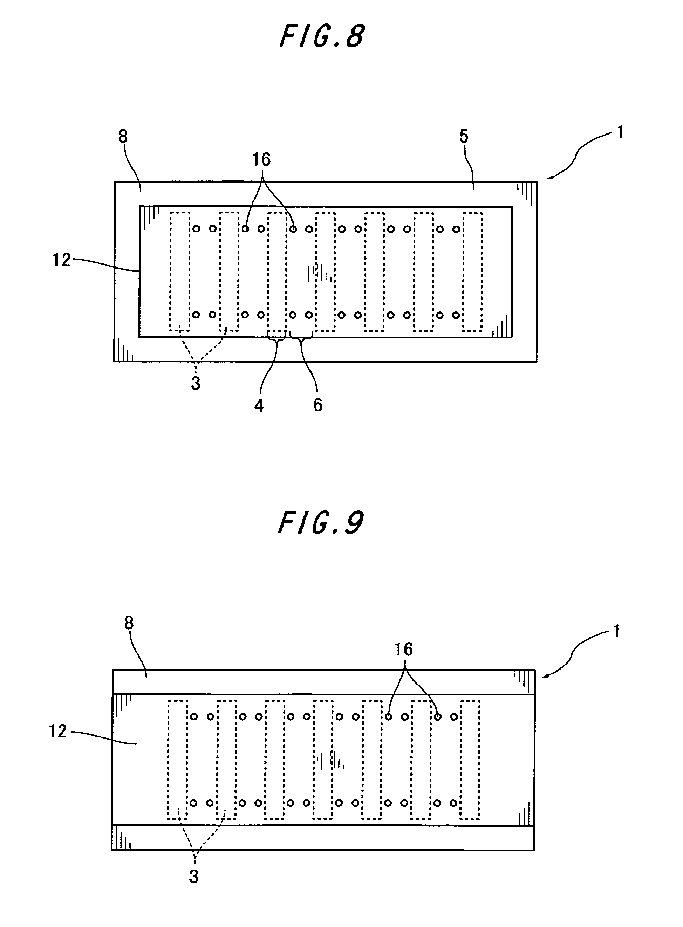

Fig. 8 shows an example of the heat generating body 1 provided with the air permeability adjusting material 12 so as to cover the whole of plural sectional exothermic parts 4. An air hole 16 is provided in the position opposite to the sectioned part 6 of the air permeability adjusting material 12. This air hole 16 works as an air intake.

Fig. 9 shows an example of the heat generating body 1 provided with the air permeability adjusting material 12 over the whole of the longitudinal direction of the heat generating body 1. An air hole 16 is provided in the position opposite to the sectioned part 6 of the air permeability 12, and this air hole 16 works as an air intake.

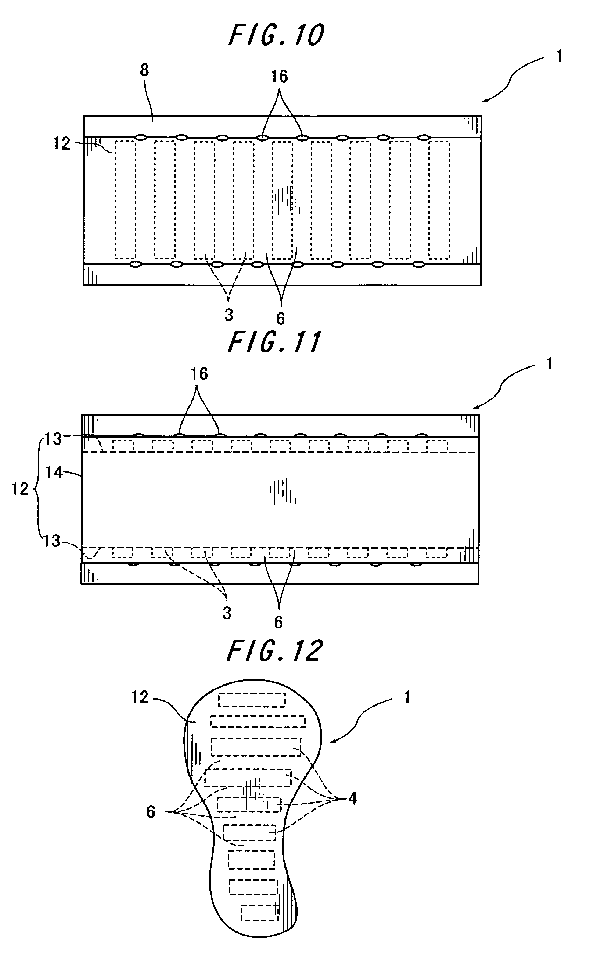

Fig. 10 shows an example of the heat generating body 1 provided with the air permeability adjusting material 12 over the whole of the longitudinal direction of the heat generating body 1. An air intake is formed in both end parts of a space which is constituted of the air permeability adjusting material 12 and the sectioned part 6.

Fig. 11 shows an example of the heat generating body 1 provided with the air permeability adjusting material 12 over the whole of the longitudinal direction of the heat generating body 1. In this example, both end parts 13 of the air permeability adjusting material 12 are not stuck, and a central part 14 is permeable to air. Furthermore, an air intake is formed in both end parts of a space which is constituted of the air permeability adjusting material 12 and the sectioned part 6.

Fig. 12 shows an entire last form heat generating body in which an air permeability adjusting material is provided over the whole surface of the heat generating body and the air permeability adjusting material is installed in a top of each sectional exothermic part via an adhesive seal part. Furthermore, a side part of the heat generating body works as an air intake.

The "air-permeable adjusting material" as referred to in the invention comprises a sectional exothermic part and a sectioned part and covers an exothermic part having a difference of altitude via an adhesive layer, etc., thereby adjusting the air permeability into the sectional exothermic part. That is, in the air permeability adjusting material, by covering the exothermic part by the air-permeable adjusting material while utilizing a difference of altitude between the sectional exothermic part and the sectioned part, a partitioned space is formed in at least a part of the periphery of the sectional exothermic part, thereby adjusting the air permeability between the outside and the sectional exothermic part and also imparting a heat insulating effect.

The air permeability of the air permeability adjusting material is not limited so far as it is able to adjust air retention or air permeability in at least a part of the periphery of the sectional exothermic part. However, it is preferable that the air permeability of the air permeability adjusting material is lower than that on the air-permeable surface of the sectional exothermic part as a covering part for covering the heat generating composition molded body.

Furthermore, a region having higher air permeability than that of a covering part for covering the heat generating composition molded boy may be provided in a local region of the air permeability adjusting material by perforation or the like, thereby keeping the air permeability lower than that of the air-permeable surface of the sectional exothermic part in other region. Furthermore, an air intake may be provided in a portion of the air permeability adjusting material positioned in a space crossing the sectional exothermic parts. For example, a primary air intake may be provided in a region of the air permeability adjusting material corresponding to the foregoing space by entirely covering at least the air-permeable region of the exothermic part by the air permeability adjusting material.

The fixing region between the air permeability adjusting material and the exothermic part is not limited so far as the both can be fixed and air can go in and out from at least the periphery of the sectional exothermic part. However, the following can be enumerated.

1) The fixing region is fixed in the both ends of the exothermic part or heat generating body.
2) A space is provided entirely in a substantially central part of the exothermic part, and other exothermic part region is defined as the fixing region.
3) A substantially top part of each sectional exothermic part and a substantially central part of each sectioned part are defined as the fixing region.

Here, as the air permeability adjusting material, any material can be used so far as it is provided with a space which communicates with the outside in the surroundings of the sectional exothermic part. Examples of an air permeability adjusting material having a bonding layer and utilizing a plastic film include PE/adhesive, PP/adhesive, polyester/adhesive, PE/non-woven fabric/air-permeable adhesive, PE/non-woven fabric/PE/adhesive, PE/PET/M/PE/non-woven fabric/air-permeable adhesive, PE/heat seal material, PE/non-woven fabric/heat seal material, PE/non-woven fabric/PE/heat seal material, and PE/polyester/M/PE/non-woven fabric/heat seal material. Here, M represents a metal (for example, aluminum and silver), a semiconductor (for example, silicon oxide, silicon oxynitride, silicon nitride, and aluminum oxide), or a metal oxide, oxynitride or nitride. Furthermore, a portion for placing fixing means such as an adhesive layer and a heat sealing agent layer is not limited, and whether it is provided partially or entirely may be properly determined depending upon the intended purpose.
The bonding layer for fixing the air permeability adjusting material is not limited so far as the air permeability adjusting material can be fixed to the heat generating body and is constituted of a usually used bonding agent or adhesive. In particular, an adhesive is useful, and the adhesive constituting the foregoing adhesive layer can be used.
Furthermore, a method for providing the bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape. Its thickness is not particularly limited but is in the range of from 5 to 1,000 $\mu$m, preferably from 10 to 500 $\mu$m, and more preferably from 15 to 250 $\mu$m. When the thickness of the bonding layer is less than 5 $\mu$m, a desired adhesive strength may not be possibly obtained. On the other hand, when it exceeds 1,000 $\mu$m, not only it becomes bulky and becomes worse in feeling for use, but also it becomes worse in economy, and therefore, such is not preferable.
In the heat generating body, for the purposes of carrying out heat sealing at high speed, making the heat seal width thin and surely carrying out heat sealing, there can be employed heat sealing after temporary adhesion in which after temporarily adhering the substrate and the covering material via a sticky layer, heat sealing is carried out. That is, the substrate and the covering material of the air-permeable accommodating bag have a heat seal layer, a heat seal part is formed of the heat seal layer, the heat seal part is formed by heat sealing after temporary adhesion by an adhesive layer to form a temporary adhering seal, and an adhesive component which constitutes the adhesive layer and a component of the heat seal material which constitutes the heat seal layer are co-present in the heat seal part.
In the invention, as a heat seal material constituting a heat seal layer, a single raw material may be used, or a composite raw material having a heat seal layer may be used. The heat seal material is not limited so far as at least a part thereof can be welded upon heating. Examples thereof include hot melt based resins such as polyolefins (for example, polyethylene and polypropylene) or olefin copolymer resins, ethylene based hot melt resins (for example, ethylene-vinyl acetate copolymer resins and ethylene-acrylic acid ester copolymer resins (for example, ethylene-isobutyl acrylate copolymer resins)), polyamide based hot melt resins, butyral based hot melt resins, polyester based hot melt resins, polyamide based hot melt resins, polyester based hot melt resins, polymethyl methacrylate based hot melt resins, polyvinyl ether based hot melt resins, polyurethane based hot melt resins, polycarbonate based hot melt resins, such as polyvinyl acetate, and vinyl chloride-vinyl acetate copolymers; and films or sheets thereof. Furthermore, in these hot melt based resins or films or sheets thereof, ones having various additives (for example, an antioxidant) compounded therein can be used. In particular, low density polyethylene and polyethylene obtained by using a metallocene catalyst are useful.
In the case of interposing a heat generating composition
molded body between a substrate and a covering material, the "temporary adhesion" as referred to in the invention means weak pressure-sensitive bonding or adhesion for the purpose of holding the accommodated heat generating composition molded body until at least the substrate and the covering material are adhered to each other via a sticky layer made of an adhesive and heat sealed.
Furthermore, the "deadhesion" as referred to herein means that in the temporary adhering seal part after heat seal, the heat generating composition in a non-heat sealed region is transferred to the foregoing region, thereby releasing the temporary adhesion.
The temporary adhering seal part is formed via a sticky layer. An adhesive constituting the sticky layer is not limited so far as it is a layer formed of a polymer composition which is tacky at the normal temperature and can be heat sealed after the temporary adhesion.

Furthermore, although the adhesive of the foregoing adhesive layer can be used as the adhesive constituting the sticky layer to be used for the temporary adhesion, a non-hydrophilic adhesive is preferable. As the adhesive constituting the sticky layer, one which is well compatible with the heat seal material constituting the heat seal is preferable, and a melting point of a base polymer of the adhesive is preferably not higher than a melting point of the heat seal material. In particular, hot melt based adhesives are preferable. Furthermore, in the case where the heat seal material is made of an olefin based raw material, preferred examples of the adhesive include olefin based adhesives.

Incidentally, a method for providing a sticky layer for the temporary adhesion is not limited. The sticky layer may be entirely provided or partially or intermittently provided. Examples of its shape include various shapes such as a network-like shape, a stripe-like shape, a dot-like shape, and strip-like shape.

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm.

A capacity of the sectional exothermic part or a volume of the heat generating composition molded body is usually from 0.015 to 500 cm$^3$, preferably from 0.04 to 30 cm$^3$, more preferably from 0.1 to 30 cm$^3$, further preferably from 1 to 30 cm$^3$, and still further preferably from 3 to 20 cm$^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0.6 to 1, preferably from 0.7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.

Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.

Furthermore, the "volume of the heat generating composition molded body of the invention" as referred to herein means a volume of the heat generating composition molded body or compressed heat generating composition molded body.

Furthermore, the "capacity of the sectional exothermic part" as referred to herein means an internal capacity of the sectional exothermic part having a heat generating composition molded body accommodated therein.

A heat generating body in which a number of sectional exothermic parts are continuously provided and a perforation from which cutting by hand is possible is provided in the sectioned part can be cut into an appropriate size at the time of use on the basis of the purpose for use adaptive to a place for application of a human body, or the like and applied. In that case, the size of the heat generating body and the size and number of the sectional exothermic parts may be properly set up. There are no limitations regarding such size and number. Furthermore, the sectioned part can be formed in arbitrary directions such as a length or width direction, length and width directions, and an oblique direction.

The "perforation" as referred to in the invention includes one which is intermittently cut for the purpose of improving flexural properties of the sectioned part and one which is intermittently cut such that cutting by hand is possible. Its degree, length and aperture are not limited but are determined depending upon the desire. The perforation may be provided in all sectioned parts or may be partially provided. The shape is not particularly limited, and examples thereof include a circle, an ellipse, a rectangle, a square, and a cut line (linear shape). For example, in the perforation which is intermittently cut such that cutting by hand is possible, a circular hole having an aperture of from $\phi$10 to 1,200 $\mu$m can be enumerated. The aperture of the hole is more preferably from $\phi$20 to 500 $\mu$m.

It is preferable that the holes are positioned lined up in the length and width. Furthermore, a shortest space between outer peripheries of the adjacent holes in the length and width is not limited so far as it is satisfactory with flexural properties and possibility of cutting by hand. The shortest space is preferably from 10 to 2,000 $\mu$m, more preferably from 10 to 1,500 $\mu$m, further preferably from 20 to 1,000 $\mu$m, still further preferably from 20 to 500 $\mu$m, and even further preferably from 20 to 200 $\mu$m. The cutting properties by hand are remarkably improved by a balance between the aperture of the hole and the shortest space of outer peripheries of the adjacent holes in the length and width.

The hole may be a cut line, and its length may be a length corresponding to the aperture or may be larger than the aperture. A shortest space between ends of the adjacent cut lines in the length and width is corresponding to the shortest space between outer peripheries of the adjacent holes.

For example, an aperture of the hole of from $\phi 10$ to 2, 000 $\mu$m is corresponding to a length of from 10 to 2,000 $\mu$m, and a shortest space between outer peripheries of the adjacent holes in the length and width of from 10 to 2,000 $\mu$m is corresponding to a shortest space between ends of the adjacent cut lines in the length and width of from 10 to 2,000 $\mu$m. In the case of a break, since it becomes long in one direction, its length can be prolonged and may be from 10 to 50,000 $\mu$m. A shortest distance between the breaks adjacent to each other in the length and width directions may be from 1 to 5,000 $\mu$m.

The entire surface or a part of at least one member of the heat generating composition molded body, the substrate, the covering material, the air-permeable adhesive layer and the underlay material may be subjected to a pressurizing treatment or the like or may be provided with irregularities. In this way, the movement of the heat generating composition molded body between the substrate and the covering material may be prevented.

That is, a material prepared by properly compressing the heat generating composition molded body of the invention under pressure is remarkably improved in moldability. For example, even when a perforated film which is difficult with respect to the pressure adjustment is used as a raw material of an air-permeable part in place of a porous film, or even when an inner pressure of the accommodating bag becomes equal to or more than an outer pressure, shape collapse hardly occurs so that it is possible to use a perforated film. Accordingly, not only the range for selecting an air-permeable raw material is widened so that the costs can be lowered, but also a body to be warmed can be uniformly warmed at an appropriate temperature over a long period of time.

In the exothermic part, by containing a magnetic substance in at least a part thereof or one sectional exothermic part, it is possible to accommodate a magnetic substance such as a magnet for the purpose of improving circulation of the blood or improving stiffness of the shoulders due to a magnetic effect.

Further, the shape of the heat generating body is not limited but can be selected from the group consisting of a rectangular shape, a circular shape, an elliptical shape, a polygonal shape, a broad bean-like shape, an eye mask-like shape, a paper lantern-like shape, a cocoon-like shape, a gourd-like shape, a rectangular shape with rounded corners, a square shape with rounded corners, an egg-like shape, a boomerang-like shape, a comma-shaped bead-like shape, a wing-like shape, a nose-like shape, a star-like shape, and a foot-like shape.

Furthermore, the heat generating body or accommodating bag can be provided with at least one member of characters, designs, symbols, numerals, patterns, photographs, pictures, and colors in at least a part thereof.

The fixing means is not limited so far as it has capability for fixing a thermal packaging body for joint surroundings or a material having an exothermic part to a prescribed part.

As the fixing means, an adhesive layer, a hook and eye, a hook and button, a hook and loop fastener such as Velcro, a magnet, a band, a string, and combination thereof can be arbitrarily used.

Incidentally, in the case of a band, fixing means for adjustment may be further constructed by a combination of a hook and loop fastener and an adhesive layer.

Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.

The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive of the invention is classified into a non-hydrophilic adhesive, a mixed adhesive, and a hydrophilic adhesive (for example, a gel).

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, a pressure-sensitive adhesive, a non-hydrophilic adhesive, and a hydrophilic adhesive are employable.

The adhesive layer includes one layer of a non-hydrophilic adhesive constituted of the non-hydrophilic adhesive and non-hydrophilic adhesive layers constituted of the non-hydrophilic adhesive.

It is to be noted that a material whose water absorption properties are improving by containing a water absorptive polymer or a water retaining agent in the non-hydrophilic adhesive layer is dealt as the non-hydrophilic adhesive layer.

A hot melt based adhesive may be provided between the hydrophilic adhesive layer and a substrate or a covering material. Furthermore, in the case where the hydrophilic adhesive is provided in a thermal packaging body for joint surroundings, there is no limitation. After seal treating a thermal packaging body for joint surroundings, a hydrophilic adhesive layer may be provided in the thermal packaging body for joint surroundings.

Furthermore, the adhesive layer may or may not have air permeability and may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives made of, as a base polymer, an A-B-A type block copolymer (for example, SIS, SBS, SEBS, and SIPS), vinyl chloride based adhesives made of, as a base polymer, a vinyl chloride resin, polyester based adhesives made of, as a base polymer, a polyester, polyamide based adhesives made of, as a base polymer, a polyamide, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), 1,2-polybutadiene based adhesives made of, as a base polymer, 1, 2-polybutadiene, and polyurethane based adhesives made of, as a base polymer, polyurethane; adhesives made of a modified body of the foregoing adhesive whose adhesion is improved or whose stability is changed; and mixtures of two or more kinds of these adhesives. Adhesive layers constituted of a foamed adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptane, hexene, and octene.

The "aromatic hot melt based adhesive" as referred to herein is a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound A such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS), and mixtures thereof.

As a countermeasure for preventing a lowering of adhesive strength caused due to an increase of water of the non-hydrophilic adhesive layer, an adhesive layer obtained by further compounding a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, soft liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be used as an arbitrary combination.

A temporary adhering seal part is formed via a sticky layer. An adhesive which constitutes the sticky layer is a layer formed of a polymer composition which is tacky at the normal temperature and is not limited so far as it can be heat sealed after temporary adhesion.

Furthermore, the foregoing adhesives of the sticky layer can be used as the adhesive which constitutes the sticky layer as used for temporary adhesion. Of these, non-hydrophilic adhesives are preferable. With respect to the adhesive constituting the adhesive layer, it is preferable that the adhesive is well compatible with a heat seal material constituting a heat seal and that a melting point of the base polymer of the adhesive is not higher than a melting point of the heat seal material. Hot melt based adhesives are especially preferable for hot melt based bonding agents. Furthermore, in the case where the heat seal material is an olefin based raw material, preferred examples thereof include olefin based adhesives.

A bonding layer for fixing the air permeability adjusting material is constituted of a bonding agent or an adhesive which is usually used. In particular, an adhesive is useful, and the foregoing adhesives for constituting the adhesive layer can be used.

Furthermore, a method for providing a bonding layer is not limited so far as the air permeability adjusting material can be fixed. The bonding layer may be entirely provided or partially or intermittently provided. Examples of its shape include various shapes such as a network-like shape, a stripe-like shape, a dot-like shape, and strip-like shape.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing in-conveniences against the both. In particular, the transfer of water occurs during the storage. In order to prevent this, it is preferable that the packaging material present therebetween at least has a moisture permeability of not more than 2 $g/m^2/day$ in terms of a moisture permeability according to the Lyssy method. By using this, in the case where the heat generating body is accommodated in an outer bag as an air-impermeable accommodating bag and stored, the transfer of water can be prevented.

In the case where a hydrophilic adhesive layer is used as the adhesive layer, the moisture permeability of a moisture-proof packaging material provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2/day$, preferably not more than 1.0 $g/m^2/day$, more preferably not more than 0.5 $g/m^2/day$, and further preferably from 0.01 to 0.5 $g/m^2/day$. These values are a value under a condition under an atmospheric pressure at 40 °C and 90 % RH. Incidentally, the moisture-proof packaging material can be used as a substrate or a covering material and may be laminated singly on a substrate, a covering material, or the like.

The moisture-proof packaging material is not limited so far as the transfer of water between the heat generating composition molded body and the hydrophilic adhesive layer can be prevented. Examples thereof include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used. Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

The heat generating body is accommodated in an air-tight air-impermeable accommodating bag, stored and transported. Examples thereof include a heat generating body prepared by interposing a produced heat generating body between two sheets of an air-impermeable film or sheet, punching the two sheets of film or sheet into a size larger than that of the heat generating body at the same time with or after this interposition, and sealing the two sheets of film or sheet in the surroundings exceeding the size of the heat generating body at the same time with or after this punching. The outer bag is not limited so far as it is air-impermeable and may be made of a laminate. Usually, an outer bag prepared from an air-impermeable raw material is used.

Usually, the accommodating bag of the invention is made of a substrate and a covering material, and in addition, an underlay material may be provided between the substrate and the covering material. The substrate is substantially planar and does not have an accommodating pocket; the covering material covers the heat generating composition provided on the substrate; the sectional exothermic part which is constituted by heat sealing the periphery of the heat generating composition is made of two or more plural sectional exothermic parts; the respective exothermic parts are disposed at intervals by the sectioned part which is a heat seal part; and the exothermic part is formed of a gathering of the foregoing sectional exothermic parts. Here, in the invention, the substrate and the covering material are not distinguished from each other depending upon a raw material constitution; but a raw material on which the heat generating composition molded body is laminated is defined as a substrate, and a raw material which is then covered on the substrate or heat generating composition molded body is defined as a covering material.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable raw materials, and heat sealable raw materials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example. That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons); and examples of the layer H include non-water absorptive papers and water absorptive papers. Examples of the substrate or covering material include heat seal layer made of polyethylene obtained by using a metallocene catalyst/polypropylene film, polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/(paper and/or porous film) /perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10,000 $g/m^2/24$ hr, preferably from 70 to 5,000 $g/m^2/24$ hr, more preferably from 100 to 2, 000 $g/m^2/24$ hr, and further preferably from 100 to 700 $g/m^2/24$ hr in terms of moisture permeability by the Lyssy method.

When the moisture permeability is less 50 $g/m^2/24$ hr, the heat value is small and a sufficient thermal effect is not obtained, and therefore, such is not preferable. On the other hand, when it exceeds 10,000 $g/m^2/24$ hr, the exothermic temperature is high so that a problem in safety may possibly be generated, and therefore, such is not preferable. However, there is no limitation even when the moisture permeability exceeds 10,000 $g/m^2/24$ hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials, films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

The outer bag is not limited so far as it is air-impermeable and may be made of a laminate. Examples thereof include nylon, polyester and polypropylene films which are subjected to a moisture-proof treatment with OPP, CPP, polyvinylidene chloride, metal oxides (including semiconductors) such as aluminum oxide and silicon oxide, etc., aluminum foils, and aluminum-deposited plastic films. Examples include a heat generating body in which the produced heat generating body is sealed between two air-impermeable films or sheets.

The heat generating composition is not particularly limited so far as it is able to cause heat generation upon contact with air. Examples thereof include a heat generating composition which contains, as essential components, an iron powder, a carbon component, a reaction accelerator and water but does not contain a flocculant aid, a flocculant, an agglomeration aid, a dry binding material, a dry binding agent, a dry binder, an adhesive binder, a thickener and an excipient, contains surplus water so as to have a water mobility value of from 0.01 to 20 and has moldability due to the surplus water, with the water in the heat generating composition not functioning as a barrier layer, and is capable of causing a heat reaction upon contact with air.

In addition, if desired, at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be further added to the heat generating composition.

Furthermore, in the heat generating composition of the invention or the like, although there is no particular limitation for the compounding ratio thereof, it is preferred to select the compounding ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the water absorptive polymer is from 0.01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in compounding ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0.01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight based on 100 parts by weight of the iron powder. Incidentally, a magnetic material may further be compounded, and its compounding ratio may be properly determined depending upon the desire.

Incidentally, these compounding ratios can also be applied in a reaction mixture and a heat generating mixture. Furthermore, a water mobility value of the reaction mixture is usually less than 0.01.

As the water, one from a proper source may be employed. Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth) acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly(meth)acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, $NaOH$, $KOH$, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

Examples of the oxidizing agent include nitrates, oxides, peroxides, halogenated oxygen acid salts, permanganates, and chromates.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol-ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

As the "iron powder" as referred to herein, usual iron powders, iron alloy powders and active iron powders such as iron powders comprising particles, a surface of each of which is at least partially covered with an oxygen-containing film , and iron alloy powders comprising particles , a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed.

The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;

(B) an active iron powder in which the content of wustite is from 2 to 50 % by weight in terms of an X-ray peak intensity ratio to iron;

(C) an iron powder having an iron oxide film having a thickness of 3 nm or more on the surface thereof; and

(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 $\mu$m, more preferably from 30 nm to 100 $\mu$m, further preferably from 30 nm to 50 $\mu$m, still further preferably from 30 nm to 1 $\mu$m, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 $\mu$m or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0.5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.

Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.

The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.

Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;

(2) a process for producing a heat generating mixture by subjecting a reaction mixture of an iron powder, a reaction accelerator, an acidic substance and water in an oxidizing gas atmosphere to a self-exothermic reaction;

(3) a process for producing a heat generating mixture by subjecting a reaction mixture of an iron powder, a reaction accelerator, a carbon component and water in an oxidizing gas atmosphere to a self-exothermic reaction;

(4) a process for producing a heat generating mixture by subjecting a reaction mixture of an iron powder, a reaction accelerator, an acidic substance, a carbon component and water in an oxidizing gas atmosphere to a self-exothermic reaction;

(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;

(6) a process for producing a heat generating mixture by carrying out the method as set forth above in any one of (1) to (5) under circumstances heated so as to have temperature of at least 10 °C higher than the circumferential temperature;

(7) a process for producing a heat generating mixture by carrying out the method as set forth above in any one of (1) to (6) by blowing an oxidizing gas;

(8) a process for producing a heat generating mixture by carrying out the method as set forth above in (7) by blowing the oxidizing gas heated so as to have a temperature of at least 10 °C higher than the circumferential temperature;

(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;

(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;

(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and

(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.

Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.

Furthermore, the contact treatment with an oxidizing gas may be carried out with or without stirring in a fluidized or non-fluidized state and may be carried out in a batch or continuous system.

Examples of the final heat generating composition include:

1) a heat generating composition containing, as a heat generating composition raw material, a heat generating mixture produced in the process as set forth above in any one of (1) to (12);

2) a heat generating composition obtained by adding other components to the heat generating composition as set forth above in 1); and

3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1,000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.

2) A magnet is provided in the vicinity of a central part of the back side of a polyvinyl chloride-made supporting plate (3 mm in thickness × 600 mm in length × 600 mm in width) of a footed supporting table so as to cover a cavity shape of a molding die.

3) A temperature sensor is placed on the central part of the supporting plate.

4) A polyethylene film (25 $\mu$m in thickness × 250 mm in length × 200 mm in width) as provided with an adhesive layer having a thickness of about 80 $\mu$m is stuck onto the supporting plate via a sticky layer such that the center of the polyethylene film is positioned at the sensor.

5) The heat generating composition is taken out from the outer bag.

6) A template (250 mm in length × 200 mm in width) having a cavity (80 mm in length × 50 mm in width × 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.
The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.
In the iron powder of the invention, not only a region where (1) entire (uniform) corrosion, (2) pitting or crevice corrosion, (3) stress corrosion cracking, or the like is generated, but also irregularities or crevices are formed. For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.
The thickness of the oxygen-containing film of a prescribed amount or the oxygen-containing film of iron powder containing wustite and the amount of wustite are applied to the heat generating composition or the heat generating composition molded body at the time of lamination.

An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.
Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50.

The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.

2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.

3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

Furthermore, so far as the rising characteristics are not affected, the heat generating composition having a water mobility value falling outside the range of from 0.01 to 20 can contain a water-soluble polymer, a flocculant aid, a flocculant, an agglomeration aid, a dry binding material, a dry binding agent, a dry binder, an adhesive raw material, a tackifier, an excipient, a flocculating agent, or a soluble sticky raw material.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably

not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

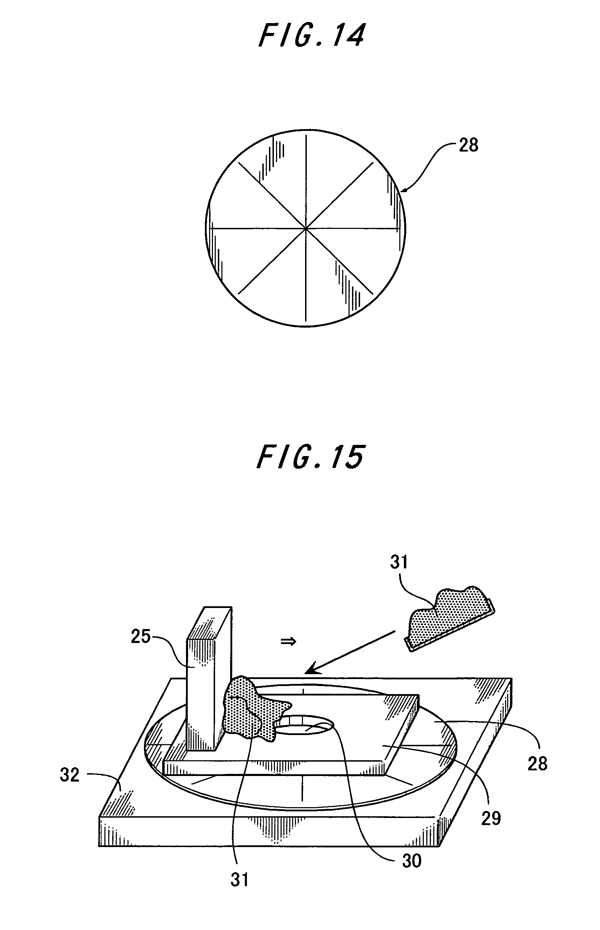

The "water mobility value" as referred to herein is a value showing an amount of surplus water which can transfer to the outside of the heat generating composition in water present in the heat generating composition. This water mobility value will be described below with reference to Figs. 14 to 18.

As shown in Fig. 14, a filter paper 28 of No. 2 (second class of JIS P3801) in which eight lines are drawn radiating from the central point with an interval of 45° is placed on a stainless steel plate 32 as shown in Figs. 15 and 16; a template 29 having a size of 150 mm in length $\times$ 100 mm in width and having a hollow cylindrical hole 30 having a size of 20 mm in inner diameter $\times$ 8 mm in height is placed in the center of the filter paper 28; a sample 31 is placed in the vicinity of the hollow cylindrical hole 30; and a stuffer plate 25 is moved on and along the template 29 and inserted into the hollow cylindrical hole 30 while stuffing the sample 31, thereby leveling the sample (force-in die molding).

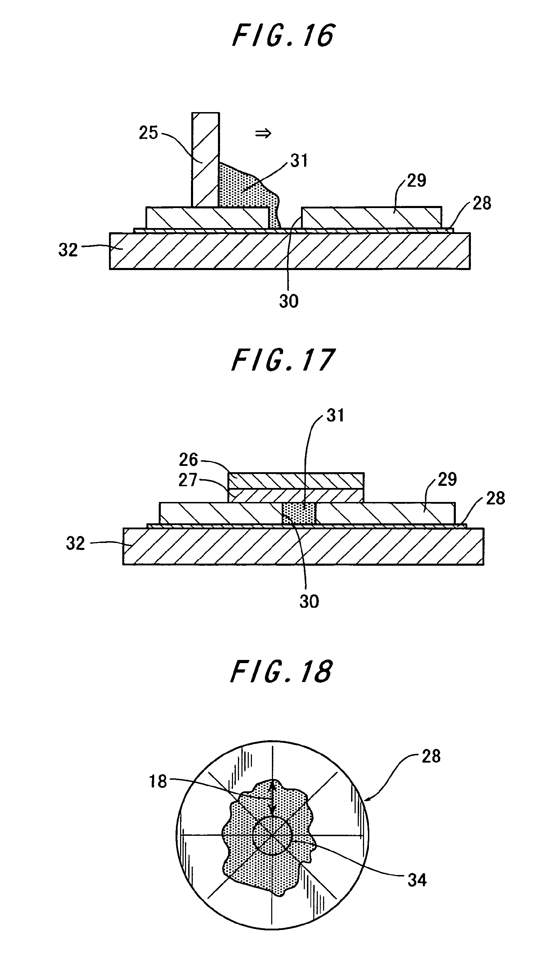

Next, as shown in Fig. 17, a non-water absorptive 70 $\mu$m-thick polyethylene film 27 is placed so as to cover the hole 30, and a flat plate 26 made of stainless steel having a size of 5 mm in thickness $\times$ 150 mm in length $\times$ 150 mm in width is further placed thereon and held for 5 minutes such that an exothermic reaction is not caused.

Thereafter, a shown in Fig. 18, the filter paper 28 is taken out, and an oozed-out locus of the water or aqueous solution is read as a distance 33 (unit: mm) from a periphery 34 as an edge of the hollow cylindrical hole to an oozed-out tip along the radiating lines. Similarly, a distance 33 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter $\times$ 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

```
(Water mobility value) = {[Water content value (mm)]/

[(Real water content value (mm))] × 100
```

With respect to the same sample, five points are measured, and the five water mobility values are averaged, thereby defining an average value thereof as a water mobility value of the sample.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

A heat generating composition having a water mobility value of less than 0.01 is insufficient in moldability. A heat generating composition having a water mobility value of from 0.01 to 50 has moldability and therefore, is a moldable heat generating composition. When the water mobility value exceeds 20, it is necessary that a part of water of the heat generating composition is removed by water absorption, dehydration, etc. That is, unless a part of water in the heat generating composition molded body is removed by water absorption, dehydration, etc. using a water absorptive packaging material, etc., a practical useful exothermic reaction is not caused. Incidentally, in the case where a water absorptive polymer having a low water absorption speed is used and although a high water mobility value is exhibited at the time of molding, after elapsing a certain period of time, a part of surplus water is taken in the water absorptive polymer, whereby the heat generating composition becomes in an exothermic state with a water mobility value of from 0.01 to 20, even a heat generating composition having a high water mobility value is dealt as a heat generating composition in which surplus water does not function as a barrier layer. In a heat generating composition having a water mobility value exceeding 50, surplus water is too much, the heat generating composition becomes in a slurry state and loses moldability, and the surplus water functions as a barrier layer. Thus, even upon contact with air as it is, an exothermic reaction is not caused.

Furthermore, the "water mobility value" as referred to herein is a value obtained by digitizing surplus water which is the water content capable of being easily and freely oozed out the system in water which is contained in the heat generating composition or mixture or the like. In a mixture in which some components of the heat generating composition or mixture or the like are mixed, the amount of the surplus water is variously changed depending the amount of a component having a water retaining ability such as a water retaining agent, a carbon component and a water absorptive polymer and wettability of each component, and therefore, it is every difficult to predict the water mobility value from the amount of addition of water. Accordingly, since the amount of surplus water of the heat generating composition or mixture of the like is determined from the water mobility value, by determining the amount of addition of water and the amount of other components, a heat generating composition or mixture or the like having a substantially fixed amount of surplus water is obtained with good reproducibility. That is, by previously examining the water mobility value and a composition ratio of a heat generating composition or mixture or the like, a heat generating composition or mixture or the like as compounded along that composition ratio has a water mobility value falling within a fixed range, namely, an amount of surplus water falling within a fixed range. Thus, it is possible to easily produce a variety of heat generating compositions such as a powdered heat generating composition which causes heat generation upon contact with air but does not have moldability, a heat generating composition which causes heat generation upon contact with air and has moldability, and a heat generating composition which, after discharging out a fixed amount of surplus water from the system by water absorption, etc., causes heat generation upon contact with air and has moldability. Accordingly, if the water mobility value is known, it is possible to note what state does the subject heat generating composition or mixture or the like take.

If the water mobility value is employed, it is possible to embody a desired state with good reproducibility by a simple measurement. Thus, it becomes possible to determine a component ratio of the heat generating composition on the basis of the water mobility value obtained by the measurement and the component ratio, thereby simply achieving actual production of a heat generating composition.

As a use example of the water mobility value, water (or a reaction accelerator aqueous solution) is added to and mixed with a mixture of specified amounts of heat generating composition components exclusive of water (or a reaction accelerator aqueous solution), thereby producing plural heat generating compositions having a different water content. Next, a water mobility value of each of the heat generating compositions is measured, thereby determining a relationship between the amount of addition of water (or a reaction accelerator aqueous solution) and a water mobility value.

A heat generating composition which has moldability and causes heat generation upon contact with air has a water mobility value of from 0.01 to 20. By determining a compounding ratio of the respective components therefrom to prepare a mixture in this compound ratio, a moldable heat generating composition in which water does not function as a barrier layer and which has moldability causes heat generation upon contact with air can be produced with good reproducibility. In this way, since surplus water is used as a connecting substance and a flocculant aid or a dry binding material is not used, reaction efficiency of the iron powder does not drop. Thus, an exothermic performance can be obtained in a small amount as compared with the case of using a flocculant aid or a dry binding material.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

By using a moldable heat generating composition containing this surplus water as a connecting substance, it becomes possible to produce, for example, a super thin and super flexible heat generating body having plural sectional exothermic parts of a heat generating composition molded body on a substantially planar substrate in a maximum width of preferably from 1 to 50 mm, and more preferably from 1 to 20 mm, or in a maximum diameter of preferably from 1 to 50 mm, and more preferably from 1 to 20 mm (in the case where two or more axes are present as in an ellipse, the major axis is dealt as a length, while the minor axis is dealt as a width).

The "surplus water" as referred to herein means water or an aqueous solution portion which is present excessively in the heat generating composition and easily transfers to the outside of the heat generating composition. The surplus water is defined as a water mobility value which is a value of water or a value of an aqueous solution portion sucked out from the heat generating composition, etc. by a filter paper. When the heat generating composition has an appropriate amount of surplus water, it is assumed that the surplus water causes hydration against hydrophilic groups in the components of the heat generating composition due to a bipolar mutual action or hydrogen bond, etc. and that it is present even in the surroundings of hydrophobic groups while having high structural properties.

This is connecting water as a connecting substance in some meaning. Besides, there is water in a state called as free water which can freely move. When the surplus water increases, the structure is softened, and the free water is found.

The "moldability" as referred to in the invention exhibits that a molded body of the heat generating composition having a cavity or concave die shape is formed by force-through molding using a trimming die having a cavity or cast molding using a concave die, whereby after molding including mold release, the molding shape of the heat generating composition molded body is held.

When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, the sealing can be achieved without causing cutting in seal. The presence of the spots causes insufficient sealing.

1) Measurement device:

With respect to the measurement device, a stainless steel-made molding die (a plate having a size of 2 mm in thickness $\times$ 200 mm in length $\times$ 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length $\times$ 24 mm in width are disposed in parallel) are disposed under the endless belt.

The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness $\times$ 200 mm in length x 200 mm in width is placed on the endless belt of the measurement device, a polyethylene film having a size of 70 $\mu$m in thickness $\times$ 200 mm in length $\times$ 200 mm in width is placed thereon, and a stainless steel-made molding die is further placed thereon.

Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1. 8 m/min. After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability.

The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (3 mm in thickness × 600 mm in length × 600 mm in width) of a footed supporting table so as to cover a cavity shape of a molding die.

2) A temperature sensor is placed on the central part the surface of the supporting plate.

3) A polyethylene film (25 $\mu$m in thickness × 250 mm in length × 200 mm in width) as provided with an adhesive layer having a thickness of about 80 $\mu$m is stuck onto the supporting plate via a sticky layer such that the center of the polyethylene film is positioned at the sensor.

4) On an underlay plate (280 mm in length × 150 mm in width x 50 $\mu$m to 2 mm in thickness), a polyethylene film (230 mm in length × 155 mm in width × 25 $\mu$m to 100 $\mu$m in thickness) is placed such that one end of the polyethylene film is projected by about 20 mm outside the underlay plate and that one end thereof in the length direction is substantially coincident with one end of the underlay plate.

5) A template (230 mm in length × 120 mm in width × 3 mm in thickness) having a cavity (80 mm in length × 50 mm in width x 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:
1) to 6) are the same as in the case of the heat generating composition molded body.

8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.

9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.

The thickness after compression is preferably from 50 to 99.5 %, more preferably from 60 to 99.5 %, and further preferably from 60 to 95 % of the die thickness.

Incidentally, in the invention, it is to be noted that the heat generating composition molded body includes a heat generating composition compressed body.

The heat generating body of the invention is able to give various shapes, thicknesses and temperature zones and therefore, can be used for various utilities such as use for a joint, facial esthetic use, use for eyes, slimming use, use for heating or warming a dripping solution, use for a wet compress pack, use for a medical body warmer, use for a neck, use for a waist, use for a mask, use for a glove, use for hemorrhage, use for relaxation of symptoms such as shoulder pain, muscular pain, and menstrual pain, use for a cushion, use for heating or warming a human body during the operation, use for a thermal sheet, use for thermally volatilizing an aroma, use for an abdomen, insecticidal use by thermal volatilization, and use for treating cancer in addition to common warming of a human body. In addition, the heat generating body of the invention can be used for heating or warming machines, pets, etc.

For example, in the case of using for relaxation of symptoms, the heat generating body of the invention is applied directly in a necessary site of the body or indirectly via a cloth, etc. Furthermore, in the case of using for heating or warming a human body during the operation, a method for using the heat generating body of the invention includes the following methods.

(1) The heat generating body is directly applied to a body requiring heating or warming.
(2) The heat generating body is fixed on a covering, etc. and covered on the body.
(3) The heat generating body is fixed on a cushion to be placed beneath the body, etc.
(4) The heat generating body is used as a covering or a cushion which is a product having the heat generating body provided therein in advance.
Incidentally, examples of the pain of muscles or bones include acute muscle pain, acute bone pain, acute reference pain, previous muscle pain, previous bone pain, chronic reference pain, and join pain of knee, elbow, etc.
The holding time is not limited but is preferably from 20 seconds to 24 hours, more preferably from one hour to 24 hours, and further preferably from 8 hours to 24 hours.
The holding temperature is preferably from 30 to 50 °C, more preferably from 32 to 50 °C, further preferably from 32 to 43 °C, still further preferably from 32 to 41 °C, and even further preferably from 32 to 39 °C.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The invention will be specifically described below with reference to the Examples, but it should not be construed that the invention is limited thereto.

[Brief Description of the Drawings]

[Fig. 1] is a plan view of an embodiment of the heat generating body of the invention.
[Fig. 2] is a cross-sectional view along the line Z-Z of the same.
[Fig. 3] is a cross-sectional view along the line X-X of the same.
[Fig. 4] is a cross-sectional view of other embodiment of the heat generating body of the invention.
[Fig. 5] is a cross-sectional view of other embodiment of the heat generating body of the invention.
[Fig. 6] is a plan view of other embodiment of the heat generating body of the invention.
[Fig. 7] is a plan view of other embodiment of the heat generating body of the invention?
[Fig. 8] is a plan view of other embodiment of the heat generating body of the invention.
[Fig. 9] is a plan view of other embodiment of the heat generating body of the invention.
[Fig. 10] is a plan view of other embodiment of the heat generating body of the invention.
[Fig. 11] is a plan view of other embodiment of the heat generating body of the invention.
[Fig. 12] is a plan view of other embodiment of the heat generating body of the invention.

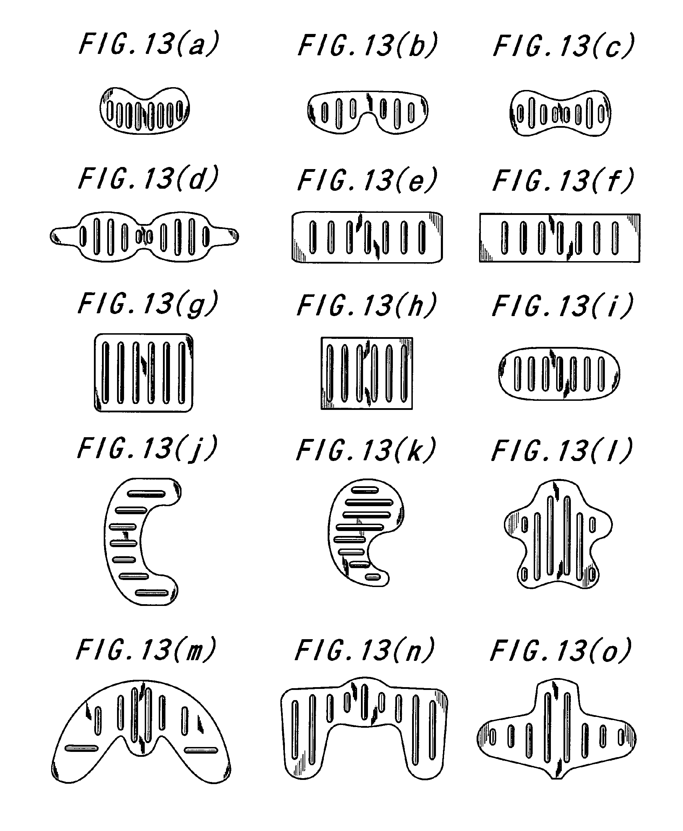

[Fig. 13] is a plan view of other embodiment of the heat generating body of the invention.

[Fig. 14] is a plan view of a filter paper for the measurement of water mobility value in the invention.

[Fig. 15] is an oblique view for explaining the measurement of water mobility value in the invention.

[Fig. 16] is a cross-sectional view for explaining the measurement of water mobility value in the invention.

[Fig. 17] is a cross-sectional view for explaining the measurement of water mobility value in the invention.

[Fig. 18] is a plan view of a filter paper after the measurement of water mobility value in the invention.

[Description of Reference Numerals and Signs]

| | |
|---|---|
| 1: | Heat generating body |
| 3: | Heat generating composition molded body |
| 4: | Sectional exothermic part |
| 5: | Covering material |
| 6: | Sectioned part |
| 7: | Perforation |
| 8: | Substrate |
| 10: | Adhesive layer |
| 11: | Separator |
| 12: | Air permeability adjusting material |
| 12a: | Adhesive seal part |
| 12b: | Non-adhesive seal part |
| 13: | Spacial part |
| 25: | Pushing plate |
| 26: | Flat plate |
| 27: | Non-water absorptive film (polyethylene film, etc.) |
| 28: | Filter paper in which eight lines are drawn radiating from the central point with an interval of 45° |
| 29: | Die plate |
| 30: | Hole |
| 31: | Sample |
| 32: | Stainless steel plate |
| 33: | Distance to the oozed-out locus of water or aqueous solution |
| 34: | Position corresponding to a hollow cylindrical hole on filter paper |

[Examples]

(Example 1)

100 parts by weight of a reduced iron powder (particle size: not more than 300 $\mu$m), 7.0 parts by weight of active carbon (particle size: not more than 300 $\mu$m), 5 parts by weight of a wood meal (particle size: not more than 300 $\mu$m), 0.8 parts by weight of a water absorptive polymer (particle size: not more than 300 $\mu$m), 0.2 parts by weight of calcium hydroxide, 0.7 parts by weight of sodium sulfite (particle size: not more than 300 $\mu$m), and 11 % salt water were mixed to obtain a heat generating composition having a water mobility value of 5. Next, twelve heat generating composition molded bodies of 5 mm in width $\times$ 60 mm in length $\times$ 2 mm in thickness were provided at intervals of 5 mm on a substrate having a separator-provided adhesive layer provided on a polyethylene film by force-through molding. Next, a covering material made of a non-woven fabric-provided polyethylene-made porous film was placed thereon such that the polyethylenes were faced at each other. The periphery of each of the heat generating composition molded bodies was heat sealed to obtain a heat generating body having an exothermic part composed of the twelve sectional exothermic parts. Incidentally, a seal width of the sectioned part between the sectional exothermic parts as a seal part was 3 mm; a width of the sectional exothermic part was 7 mm; a seal width of the surroundings of the heat generating body was 10 mm; and a difference of altitude from the sectioned part to the sectional exothermic part was about 2.5 mm. An external dimension of the heat generating body was 135 mm in length $\times$ 100 mm in width. Next, a bonding layer constituted of an adhesive was provided over the entire surface. An air permeability adjusting material made of a polyethylene film of 50 mm in width $\times$ 135 mm was stuck on the non-woven fabric while leaving both ends of the sectional exothermic part in a width of 5 mm.

This heat generating body was sealed and accommodated in an air-impermeable outer bag and allowed to stand at room temperature for 24 hours. After 24 hours, the heat generating body was taken out from the outer bag and then subjected to an exothermic test on a plate as adjusted at 30 °C. As a result, the temperature reached 35 °C within one minute, and the exothermic duration at 35 to 39 °C was long as 10 hours.

(Comparative Example 1)

A heat generating body the same as in Example 1 was prepared, except for not providing the air permeability adjusting material. This heat generating body was sealed and accommodated in an air-impermeable outer bag and allowed to stand at room temperature for 24 hours in the same manner as in Example 1. After 24 hours, the heat generating body was taken out from the outer bag and then subjected to an exothermic test in the same manner as in Example 1. As a result, the temperature reached 35 °C within one minute, and the temperature further rose to 43 °C. Thereafter, the temperature gradually dropped and reached not higher than 35 °C after a while. The exothermic duration at 35 °C or higher was short as 5 hours. Furthermore, a temperature width was large as from 35 to 43 °C.

(Example 2)

By using a heat generating body having an exothermic part made of twelve sectional exothermic parts the same as in Example 1, an air permeability adjusting material made of a polyethylene film of 50 mm in width × 135 mm and having a bonding layer constituted of an adhesive provided in the both sides thereof in a width of 10 mm was stuck on the foregoing non-woven fabric while leaving both ends of the sectional exothermic part in a width of 5 mm, thereby obtaining a heat generating body.
The peripheries of 10 mm in the both ends of the sectioned part of this heat generating body are formed as an air intake; and the air permeability adjusting material and the sectional exothermic part are not adhered in the central part of the heat generating body, whereby a spacial air-permeable layer made of a spacial part crossing the respective sectional exothermic parts is formed.
This heat generating body was sealed and accommodated in an air-impermeable outer bag and allowed to stand at room temperature for 24 hours in the same manner as in Example 1. After 24 hours, the heat generating body was taken out from the outer bag and then subjected to an exothermic test on a plate as adjusted at 30 °C. As a result, the temperature reached 35 °C within one minute, and the exothermic duration at 35 to 39 °C was long as 9 hours.

(Example 3)

A batchwise stirring tank composed of a mixer equipped with a stirring blade was used as an oxidizing gas treatment device, and air was used as an oxidizing gas. A reaction mixture consisting of 100 parts by weight of a reduced iron powder (particle size: not more than 300 μm), 5.3 parts by weight of active carbon (particle size: not more than 300 μm), 5 parts by weight of a wood meal (particle size: not more than 300 μm), 0.8 parts by weight of a water absorptive polymer (particle size: not more than 300 μm), 0.2 parts by weight of calcium hydroxide (particle size: not more than 300 μm), 0.7 parts by weight of sodium sulfite (particle size: not more than 300 μm), and 5 parts by weight of 11 % salt water and having a water mobility value of not more than 0.01 was charged in the device vessel. Next, in the state that the device vessel as adjusted at 20 °C was opened to air, the reaction mixture was treated with an oxidizing gas while stirring for 3 minutes to obtain a heat generating mixture. A temperature rise of the reaction mixture was 10 °C or higher. Next, 11 % salt water was added to the heat generating mixture to adjust the water content, thereby obtaining a heat generating composition having a water mobility value of 8. Next, twelve heat generating composition molded bodies of 5 mm in width × 60 mm in length × 2 mm in thickness were provided at intervals of 5 mm on a substrate having a separator-provided adhesive layer provided on a polyethylene film by force-through molding. Next, a covering material made of a non-woven fabric-provided polyethylene-made porous film was placed thereon such that the polyethylenes were faced at each other. The periphery of each of the heat generating composition molded bodies was heat sealed to obtain a heat generating body having an exothermic part composed of the twelve sectional exothermic parts. Incidentally, a seal width of the sectioned part between the sectional exothermic parts as a seal part was 3 mm; a width of the sectional exothermic part was 7 mm; a seal width of the surroundings of the heat generating body was 10 mm; and a height from the sectioned part to the sectional exothermic part was about 2.5 mm. An external dimension of the heat generating body was 135 mm in length × 100 mm in width. Next, an adhesive layer was provided over the entire surface. An air permeability adjusting material made of a polyethylene film of 50 mm in width × 135 mm was stuck on the non-woven fabric while leaving both ends of the sectional exothermic part in a width of 5 mm. A heat generating body in which the sectioned part is a concave, the sectional exothermic part is a convex, the sectional exothermic part is a space retaining piece-like material, the sectioned part is a spacial air-permeable layer, and the peripheries of 10 mm in the both ends of the sectioned part are formed as an air intake was prepared.
This heat generating body was sealed and accommodated in an air-impermeable outer bag and allowed to stand at room temperature for 24 hours. After 24 hours, the heat generating body was taken out from the outer bag and then subjected to an exothermic test. As a result, the temperature reached 35 °C within one minute, and the exothermic duration at 35 to 9 °C was long as 10 hours.

(Comparative Example 2)

A heat generating body the same as the heat generating body in Example 3 was prepared, except that the air permeability adjusting material was not used and that the spacial air-permeable layer was not provided. Similarly, a perforation from which cutting by hand was possible was provided in the sectioned part in every two sectional exothermic parts. This heat generating body was sealed and accommodated in an air-impermeable outer bag and allowed to stand at room temperature for 24 hours. After 24 hours, the heat generating body was taken out from the outer bag and cut off at the perforation, thereby preparing a heat generating body having two sectional exothermic parts, which was then subjected to an exothermic test. As a result, the temperature reached 40 °C within one minute, and the exothermic duration at 45 or higher was short as 2 hours. A maximum temperature was 60 °C.

(Example 4)

A batchwise stirring tank composed of a mixer equipped with a stirring blade was used as an oxidizing gas treatment device, and air was used as an oxidizing gas. 8 % salt water was mixed in a mixture consisting of 100 parts by weight of a reduced iron powder (particle size: not more than 300 $\mu$m), 2.8 parts by weight of active carbon (particle size: not more than 300 $\mu$m), 3 parts by weight of a wood meal (particle size: not more than 300 $\mu$m), 2 parts by weight of a water absorptive polymer (particle size: not more than 300 $\mu$m), 0.2 parts by weight of calcium hydroxide (particle size: not more than 300 $\mu$m), and 0.9 parts by weight of sodium sulfite (particle size: not more than 300 $\mu$m), thereby obtaining a reaction mixture having a water mobility value of not more than 0.01. Next, this reaction mixture was charged in the device vessel. Next, in the state that the device vessel as adjusted at 20 °C was opened to air, the reaction mixture was treated with an oxidizing gas while stirring for one minute to obtain a heat generating mixture. Next, 8 % salt water was added to the heat generating mixture to adjust the water content, thereby obtaining a heat generating composition having a water mobility value of 10. A temperature rise of the reaction mixture was about 20 °C. Next, twelve heat generating composition laminates of 5 mm in width $\times$ 60 mm in length $\times$ 2 mm in thickness were provided at intervals of 5 mm on a substrate having a separator-provided adhesive layer provided on a polyethylene film by force-through molding. Next, a covering material made of a non-woven fabric-provided polyethylene-made porous film was placed thereon such that the polyethylenes were faced at each other. The periphery of each of the heat generating composition molded bodies was heat sealed to obtain a heat generating body having an exothermic part composed of the twelve sectional exothermic parts. Incidentally, a seal width of the sectioned part between the sectional exothermic parts as a seal part was 3 mm; a width of the sectional exothermic part was 7 mm; a seal width of the surroundings of the heat generating body was 10 mm; and a height from the sectioned part to the sectional exothermic part was about 2.5 mm. An external dimension of the heat generating body was 135 mm in length $\times$ 100 mm in width. Next, an air permeability material having a bonding layer constituted of an adhesive provided over the entire surface thereof, made of a polyethylene film of 50 mm in width $\times$ 135 mm and having 1-mm holes 16 in a region corresponding to the vicinity of end parts of the sectioned part was stuck on the non-woven fabric so as to cover the whole of the heat generating body, thereby obtaining a heat generating body of Fig. 8.
This heat generating body was sealed and accommodated in an air-impermeable outer bag and allowed to stand at room temperature for 24 hours. After 24 hours, the heat generating body was taken out from the outer bag and then subjected to an exothermic test. As a result, the temperature reached 36 °C within one minute, and the exothermic duration at 36 to 39 °C was long as 10 hours.
Furthermore, Fig. 9 shows a plan view of a modified example of a heat generating body in which a bonding layer is not provided in the central part of the air permeability adjusting material and the central part thereof is not stuck.

(Example 5)

A heat generating body the same as the heat generating body of Example 1 was formed into a last form as illustrated in Fig. 12.
This heat generating body for foot warming was sealed and accommodated in an air-impermeable outer bag and allowed to stand at room temperature for 24 hours. After 24 hours, the heat generating body was taken out from the outer bag and then subjected to an exothermic test on a plate as adjusted at 30 °C. As a result, the temperature reached 35 °C within one minute, and the exothermic duration at 35 to 37 °C was 9 hours. Furthermore, in a deterioration promotion test at 60 °C for 30 days, the temperature reached 35 °C within one minute, and the exothermic duration at 35 to 37 °C was 7 hours. In the case where the spacial air-permeable layer was not provided, the exothermic duration was 5 hours. Thus, the deterioration at the time of preservation over a long period of time could be greatly prevented.

(Example 6)

A semi-last form heat generating body for foot warming having a half of the length of Example 5 was prepared. This semi-last form heat generating body for foot warming was sealed and accommodated in an air-impermeable outer bag and allowed to stand at room temperature for 24 hours. After 24 hours, the heat generating body was taken out from the outer bag and then subjected to an exothermic test on a plate as adjusted at 30 °C. As a result, the temperature reached 35 °C within one minute, and the exothermic duration at 35 to 37 °C was 8 hours.

(Example 7)

A semi-last form heat generating body for foot warming having a half of the length of Example 5 was prepared. This semi-last form heat generating body for foot warming was sealed and accommodated in an air-impermeable outer bag and allowed to stand at room temperature for 24 hours. After 24 hours, the heat generating body was taken out from the outer bag and then subjected to an exothermic test on a plate as adjusted at 30 °C. As a result, the temperature reached 35 °C within one minute, and the exothermic duration at 35 to 37 °C was 8 hours.

(Example 8)

Fig. 13 shows modified examples of the shape of the heat generating body of the invention.

(a) shows a broad bean-like shape; (b) shows an eye mask-like shape; (c) shows a cocoon-like shape; (d) shows a gourd-like shape; (e) shows a rectangular shape with rounded corners; (f) shows a rectangular shape; (g) shows a square shape with rounded corners; (h) shows a square shape; (i) shows an egg-like shape; (j) shows a boomerang-like shape; (k) shows a comma-shaped bead-like shape; (1) shows a star-like shape; (m) shows a wing-like shape; (n) shows a wing-like shape; and (o) shows a nose-like shape, respectively. Furthermore, while the directions of the long axes along the long sides of the rectangles of the sectional exothermic parts are parallel to each other, they may be arbitrarily set up. Also, a gathering of sectional exothermic parts in different directions may be employed. Modified shapes as modified on the basis of these basic skeletons can also be used.

**Claims**

1. A heat generating body having an exothermic part having a heat generating composition molded body which is a molded body of a heat generating composition capable of causing heat generation upon contact with air accomodated in a convex of an accommodating bag having plural irregularities by laminating the heat generating composition molded body on a substrate, covering a covering material thereon and then heat sealing the periphery of the heat generating composition molded body, **characterized in that**:

    1) the heat generating composition contains, as essential components, an exothermic substance, a carbon component, a reaction accelerator and water,
    2) the heat generating body is composed of a sectional exothermic part and a sectioned part, the sectional exothermic part accommodates the heat generating composition molded body therein, the sectioned part is a seal part, and the sectional exothermic part is disposed via the sectioned part,
    3) at least a part of the sectional exothermic part has an air-permeable surface,
    4) the air-permeable surface which comes into contact with the heat generating composition molded body is constituted of a non-water absorptive raw material,
    5) the sectional exothermic part and the sectioned part have a difference of altitude, and the sectional exothermic parts are covered with an air permeability adjusting material to forms a partitioned spacial part between the sectional exothermic parts, and
    6) the spacial part is provided with a primary air intake.

2. The heat generating body according to claim 1, **characterized in that** air permeability of the air permeability adjusting material is not more than that of the exothermic part.

3. The heat generating body according to claim 1, **characterized in that** the heat generating composition has a water mobility value of from 0.01 to 20.

4. The heat generating body according to claim 1, **characterized in that** the heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic

5. The heat generating body according to claim 1, **characterized in that** the spacial part has a spacial shape of at least one kind selected from a linear shape, a curved shape and a maze-like shape.

6. The heat generating body according to claim 1, **characterized in that** the air permeability adjusting material is an air-impermeable raw material.

7. The heat generating body according to claim 6, **characterized in that** the air permeability adjusting material is made of at least one kind selected from a thermoplastic synthetic resin film, a metal thin film-containing thermoplastic synthetic resin film, an air-impermeable multilayered structure having a laminate of a non-woven fabric and the thermoplastic synthetic resin film, a synthetic resin expanded body, and a multilayered structure containing the same.

8. The heat generating body according to claim 1, **characterized in that** the sectional exothermic parts are provided in a striped state at intervals interposing the sectioned part therebetween.

9. The heat generating body according to claim 1, **characterized in that** the air intake is present in the surroundings of the heat generating body, and air present in the external space flows into the heat generating composition via the air intake.

10. The heat generating body according to claim 1, **characterized in that** the air intake is provided by perforating the air permeability adjusting material.

11. The heat generating body according to claim 1, **characterized in that** a space crossing the sectional exothermic parts is provided between the air permeability adjusting material and the exothermic part.

12. The heat generating body according to claim 11, **characterized in that** the space crossing the sectional exothermic parts is provided in the substantially central part on the air-permeable surface.

13. The heat generating body as set forth in claim 1, **characterized in that** the sectional exothermic part has a shortest length of from 5 to 200 mm and a maximum height of from 0.1 to 10 mm, a plural number of the sectional exothermic parts are disposed at intervals, and a gathered exothermic part is formed of a gathering of the sectional exothermic parts.

14. The heat generating body according to claim 1, **characterized in that** an air-impermeable film is provided so as to cover the air intake in a state that it can be peeled away.

15. The heat generating body according to claim 1, **characterized in that** a fixing measure is provided on a part of the exposed surface of the heat generating body.

*FIG.1*

*FIG.2*

*FIG.3*

*FIG.4*

## FIG.5

## FIG.6

## FIG.7

# FIG.8

# FIG.9

## FIG.10

## FIG.11

## FIG.12

*FIG.13(a)*

*FIG.13(b)*

*FIG.13(c)*

*FIG.13(d)*

*FIG.13(e)*

*FIG.13(f)*

*FIG.13(g)*

*FIG.13(h)*

*FIG.13(i)*

*FIG.13(j)*

*FIG.13(k)*

*FIG.13(l)*

*FIG.13(m)*

*FIG.13(n)*

*FIG.13(o)*

# FIG.14

# FIG.15

# FIG.16

# FIG.17

# FIG.18

<table>
<tr><td colspan="2" align="center">**INTERNATIONAL SEARCH REPORT**</td><td>International application No.<br>PCT/JP2005/013005</td></tr>
</table>

A. CLASSIFICATION OF SUBJECT MATTER
   Int.Cl$^7$  A61F7/08, C09K5/16, F24J1/00

According to International Patent Classification (IPC) or to both national classification and IPC

B. FIELDS SEARCHED

Minimum documentation searched (classification system followed by classification symbols)
   Int.Cl$^7$  A61F7/08, C09K5/16, F24J1/00

Documentation searched other than minimum documentation to the extent that such documents are included in the fields searched
   Jitsuyo Shinan Koho          1922-1996   Jitsuyo Shinan Toroku Koho   1996-2005
   Kokai Jitsuyo Shinan Koho    1971-2005   Toroku Jitsuyo Shinan Koho   1994-2005

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2003-334212 A  (Maikoru Kabushiki Kaisha),<br>25 November, 2003 (25.11.03),<br>Full text; Fig. 2<br>(Family: none) | 1-15 |
| A | JP 2003-336042 A  (Maikoru Kabushiki Kaisha),<br>28 November, 2003 (28.11.03),<br>Full text; Figs. 1 to 16<br>& US 2004/0217325 A      & EP 1516900 A<br>& WO 2003/097764 A1      & CA 2439044 A<br>& CN 1496395 A | 1-15 |
| A | JP 2002-155273 A  (Kaoru USUI),<br>28 May, 2002 (28.05.02),<br>Full text; Figs. 1 to 14<br>(Family: none) | 1-15 |

[X] Further documents are listed in the continuation of Box C.          [ ] See patent family annex.

| | |
|---|---|
| *     Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| Date of the actual completion of the international search<br>   16 September, 2005 (16.09.05) | Date of mailing of the international search report<br>   04 October, 2005 (04.10.05) |
| Name and mailing address of the ISA/<br>   Japanese Patent Office | Authorized officer |
| Facsimile No. | Telephone No. |

Form PCT/ISA/210 (second sheet) (January 2004)

EP 1 782 776 A1

| INTERNATIONAL SEARCH REPORT | International application No. |
| --- | --- |
| | PCT/JP2005/013005 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2003-509120 A (The Procter & Gamble Co.), 11 March, 2003 (11.03.03), Full text; Figs. 1, 2 & US 6336935 B1 & EP 1212020 A & WO 2001/019302 A1 & AU 7116900 A & BR 14044 A & CN 1373649 A & CA 2381812 A & AU 768363 B | 1-15 |
| A | JP 2001-507593 A (The Procter & Gamble Co.), 12 June, 2001 (12.06.01), Full text; Figs. 1, 2 & US 6096067 A1 & US 6019782 A1 & EP 967944 A & WO 1998/029063 A1 & DE 69720280 T & NO 993243 A & CN 1254268 A & BR 9714448 A & HU 2866 A & AU 738531 B & AT 235202 T & DK 967944 T & ES 2193420 T & CA 2276479 A | 1-15 |
| A | JP 11-512954 A (The Procter & Gamble Co.), 09 November, 1999 (09.11.99), Full text; Figs. 1 to 3 & JP 3545769 B & WO 1997/049361 A1 & AU 735088 B | 1-15 |
| A | CD-ROM of the specification and drawings annexed to the request of Japanese Utility Model Application No. 5759/1992(Laid-open No. 58123/1993) (Shozo NAKAJIMA), 03 August, 1993 (03.08.93), Full text; Fig. 1 (Family: none) | 1-15 |
| A | JP 10-263002 A (Kabushiki Kaisha Genchi Kenkyusho), 06 October, 1998 (06.10.98), Full text; Figs. 1 to 14 (Family: none) | 1-15 |

Form PCT/ISA/210 (continuation of second sheet) (January 2004)

44

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000000260 A
- JP 2002200108 A
- JP 10265373 A
- JP 9087173 A
- JP 6145050 A
- JP 6199660 A
- JP 10279466 A
- JP 10182408 A

- JP 3161605 B
- JP 11508314 T
- JP 2002514104 T
- JP 2001507593 T
- JP 4293989 A
- JP 6343658 A
- JP 7194641 A